Europäisches Patentamt

European Patent Office

Office européen des brevets

(11) Veröffentlichungsnummer: **0 214 544 B1**

# (12) EUROPÄISCHE PATENTSCHRIFT

(45) Veröffentlichungstag der Patentschrift: **19.06.91**

(51) Int. Cl.⁵: **C07C 303/06**, C07C 309/47

(21) Anmeldenummer: **86111748.9**

(22) Anmeldetag: **25.08.86**

(54) Verfahren zur Herstellung von 1-Aminonaphthalin-4-sulfonsäure (Naphthionsäure).

(30) Priorität: **07.09.85 DE 3531922**

(43) Veröffentlichungstag der Anmeldung:
**18.03.87 Patentblatt 87/12**

(45) Bekanntmachung des Hinweises auf die
Patenterteilung:
**19.06.91 Patentblatt 91/25**

(84) Benannte Vertragsstaaten:
**CH DE FR GB IT LI**

(56) Entgegenhaltungen:
**EP-A- 0 052 307**
**EP-A- 0 063 271**

**METHODEN DER ORGANISCHEN CHEMIE,
(HOUBEN-WEYL), 4. Auflage, Band IX,
"Schwefel-, Selen-, Tellur-Verbindungen,
1955, Seiten 494-495, Georg Thieme Verlag,
Stuttgart, DE**

(73) Patentinhaber: **BAYER AG**

**W-5090 Leverkusen 1 Bayerwerk(DE)**

(72) Erfinder: **Streicher, Willi, Dr.**
**Andreas-Gryphius-Strasse 7**
**W-5000 Köln 80(DE)**
Erfinder: **Marzolph, Gerhard, Dr.**
**Semmelweisstrasse 87a**
**W-5000 Köln 80(DE)**
Erfinder: **Behre, Horst, Dr.**
**Zur alten Linde 12**
**W-5068 Odenthal(DE)**
Erfinder: **Blank, Heinz Ulrich, Dr.**
**Am Geusfelde 35**
**W-5068 Odenthal(DE)**

**Beschreibung**

Die Erfindung betrifft ein neues Verfahren zur Herstellung von 1-Aminonaphthalin-4-sulfonsäure (Napthionsäure).

Naphthionsäure ist ein wichtiges Zwischenprodukt für die Herstellung von Farbstoffen (siehe z.B. die Angaben in Ullmanns Enzyklopädie der Technischen Chemie, 4. Auflage, 1979, Band 17, S. 109). Bislang wird Naphthionsäure nach dem sogenannten Backverfahren durch Erhitzen von 1-Naphthylamin-hydrogensulfat, gegebenenfalls in einem inerten organischen Lösungsmittel, hergestellt (siehe EP-A- 0 063 271 und Ullmann loc. cit.). Dieses Verfahren liefert Naphthionsäure in hohen Ausbeuten und guter Reinheit, hat aber den Nachteil, daß es zu seiner erfolgreichen Durchführung einen erheblichen technischen Aufwand erfordert. Versuche, Naphthionsäure durch Sulfonierung von 1-Aminonaphthalin in überschüssiger Schwefelsäure herzustellen, haben bislang nur zu unbefriedigenden Ergebnissen geführt, weil bei dieser Sulfonierung Isomerengemische erhalten werden, die außer der gewünschten Naphthionsäure auch 1-Aminonaphthalin-5-sulfonsäure und 1-Aminonaphthanlin-6-sulfonsäure enthalten. Aus diesen Isomerengemischen ist die reine Naphthionsäure mittels aufwendiger und verlustreicher verfahren isolierbar (siehe z.B. die Angaben in Ann. Band 275 (1893), S. 192-207).

Überraschenderweise wurde nun gefunden, daß man 1-Aminonaphthalin-4-sulfonsäure in ausgezeichneten Ausbeuten und hoher Selektivität durch Sulfonieren von 1-Aminonaphthalin mit einem hohen Überschuß Schwefelsäure bei erhöhter Temperatur erhalten kann, wenn man die Sulfonierung in Gegenwart von Zusatzstoffen aus der Reihe Säureamide oder Alkali-, Erdalkali- oder Ammonium-sulfate, -bisulfate oder Alkali-, Erdalkali-oder Ammoniumsalze solcher Säuren, die durch Schwefelsäure aus ihren Salzen verdrängt werden, vornimmt. Die Gegenwart dieser Stoffe bewirkt, daß 1-Aminonaphthalin mit hoher Selektivität in 4-Stellung sulfoniert wird.

Als Säureamide kommen beispielsweise Carbonsäureamide, Sulfonsäureamide oder Amide der Kohlensäure in Betracht. Als Carbonsäureamide seien vor allem Formamid, Acetamid, Dimethylformamid genannt. Als Sulfonsäureamide seien vor allem Methansulfonsäureamid, Benzolsulfonsäureamid und Amidosulfonsäure genannt. Als Amide der Kohlensäure kommen vor allem Harnstoff, Alkylharnstoffe und Carbamidsäurealkyl- und -arylester in Betracht.

Als Alkali- Erdalkali- oder Ammonium-Sulfate und -Bisulfate seien vor allem Natrium-, Kalium- und Ammonium-sulfat und -Bisulfat genannt. Alkali-, Erdalkali- und Ammonium-Salze von Säuren, die durch Schwefelsäure aus ihren Salzen verdrängt werden, sind vor allem die Alkali-, Erdalkali- und Ammonium-Salze der Halogenwasserstoffsäuren, der schwefeligen Säure, der Kohlensäure, der Borsäure oder der Phosphorsäure zu verstehen, ferner niederer aliphatischer Mono- und Dicarbonsäuren wie der Ameisensäure, der Essigsäure, der Propionsäure oder aber von Sulfonsäuren wie der Naphthionsäure.

Die Ammoniumionen können sich vom Ammoniak, aber auch von primären, sekundären oder tertiären Aminen ableiten oder quarternäre Ammoniumionen sein. Die Ammoniumsalze können in situ im Sulfonierungsgemsich durch Zugabe von Ammoniak oder den entsprechenden Aminen zum Sulfonierungsgemisch hergestellt werden.

Die erfindungsgemäß bei der Sulfonierung von 1-Aminonaphthalin zu verwendenden Zusatzstoffe, z.B. Ammoniumsulfat, sind zwar schon bei der Sulfonierung anderer Naphthalinverbindungen verwendet worden (s. Houben-Weyl, Methoden der organischen Chemie, 4. Auflage, Band IX, S. 494-495: N.N. Woroshzow, Grundlagen der Synthese von Zwischenprodukten und Farbstoffen, 1966, S. 48 und 63). Trotzdem ist ihre Wirkung bei der Sulfonierung von 1-Aminonaphthalin überraschend. Es war in keiner Weise aus den vorbeschriebenen Reaktionen vorhersehbar, daß die Zusatzstoffe eine selektive Sulfonierung der 4-Stellung des 1-Aminonaphthalins bewirken könnten.

Die erfindungsgemäß zu verwendenden Zusatzstoffe werden bei der Sulfonierung in einer Menge von 0,1 bis 5 Äquivalenten (bei Verwendung von Salzen), bevorzugt 0,5 bis 3 Äquivalenten, besonders bevorzugt 1 bis 2 Äquivalenten oder 0,5 bis 4 mol (bei Verwendung der Säureamide), bevorzugt 0,8 bis 3 mol, besonders bevorzugt 1 bis 2 mol je Mol 1-Aminonaphthalin angewendet.

Die erfindungsgemäße Sulfonierung des 1-Aminonaphthalins wird bei Temperaturen von 40°C bis 160°C, bevorzugt von 60 bis 140°C vorgenommen.

Als Sulfonierungsmittel dient in dem erfindungsgemäßen Verfahren 85 bis 100 gew.-%ige Schwefelsäure. Die Schwefelsäure wird in einer Menge von 3,0 bis 10,0 mol, vorzugsweise 4 bis 8 mol je Mol 1-Aminonaphthalin angewendet.

Das als Ausgangsverbindung für die erfindungsgemäße Sulfonierung einzusetzende 1-Aminonaphthalin kann in fester Form, als Schmelze oder aber auch in Form seiner Salze, beispielsweise als Naphthylammoniumhydrogensulfat, eingesetzt werden.

Die erfindungsgemäße Sulfonierung des 1-Aminonaphthalins kann auf verschiedene Weise vorgenom-

EP 0 214 544 B1

men werden:

z.B. indem man die vorgesehene Menge an Schwefelsäure mit der vorgesehenen Menge an Zusatzstoffen vermischt, dieses Gemisch mit dem 1-Aminonaphthalin versetzt und anschließend stufenlos oder stufenweise auf die vorgesehene Sulfonierungstemperatur erwärmt und die Sulfonierung bei dieser Temperatur zu Ende führt.

In einer anderen Variante wird die vorgesehene Menge an Schwefelsäure mit der vorgesehenen Menge an Zusatzstoffen versetzt, das Gemisch langsam mit dem 1-Aminonaphthalin versetzt und während der Zudosierung des 1-Aminonaphthalins die Temperatur des Reaktionsgemisches stufenlos oder stufenweise auf die vorgesehene Reaktionstemperatur erhöht und die Sulfonierung bei dieser Temperatur zu Ende führt.

In einer anderen Variante wird nur ein Teil der vorgesehenen Schwefelsäuremenge mit der vorgesehenen Menge an Zusatzstoffen vermischt und auf die gewünschte Reaktionstemperatur erwärmt. Dann werden gleichzeitig 1-Aminonaphthalin und die restliche Schwefelsäuremenge z.B. in Form von 100 gew.-%iger Schwefelsäure oder aber auch in Form von Oleum zudosiert. Durch die Verwendung von Oleum läßt sich eine definierte Schwefelsäurekonzentration im Reaktionsgemisch aufrechterhalten.

Das Reaktionsgemisch kann nach Reaktionsende in bekannter Weise aufgearbeitet werden (s. Houben-Weyl, Methoden der organischen Chemie, 4. Auflage, Band IX, S. 435 bis 445), beispielsweise durch Verdünnen des Reaktionsgemisches mit Wasser und Absaugen des Niederschlages. Der Niederschlag wird anschließend mit Wasser säurefrei gewaschen.

Es ist auch möglich, das Reaktionsgemisch, gegebenenfalls nach Verdünnen mit Wasser, mit Calciumcarbonat und/oder Calciumoxid/hydroxid zu neutralisieren und das entstandene Calciumsulfat abzusaugen. Aus dem Filtrat kann die 1-Aminonaphthalin-4-sulfonsäure durch Ansäuern mit anorganischen Säuren, beispielsweise Salzsäure, als freie Säure ausgefällt werden.

Gegenstand der Erfindung ist also ein Verfahren zur Herstellung von 1-Aminonaphthalin-4-sulfonsäure durch Sulfonieren von 1-Aminonaphthalin, dadurch gekennzeichnet, daß man 1-Aminonaphthalin mit 3 bis 10 Mol 85-100%iger Schwefelsäure je Mol 1-Aminonaphthalin bei Temperaturen von Säureamiden oder von Alkali-, Erdalkali- oder Ammonium-sulfaten, -bisulfaten oder Alkali-, Erdalkali- oder Ammoniumsalzen von solchen Säuren vornimmt, die in Schwefelsäure aus ihren Salzen verdrängt werden, wobei man die Säureamide in einer Menge von 0,5-4 Mol je Mol 1-Aminonaphthalin und die Salze in einer Menge von 0,1-5 Äquivalenten je Mol 1-Aminonaphthalin anwendet.

Beispiel 1

In einer Mischung aus 561,5 g 96,0 %ige Schwefelsäure (5,5 Mol) und 132,0 g Ammoniumsulfat (1,0 Mol) werden bei 25° C unter Kühlen 143,0 g 1-Aminonaphthalin (1,0 Mol) in fester Form eingetragen. Die Reaktionsmischung wird anschließend in 30 Min. auf 130° C erwärmt und bei dieser Temperatur bis zum Reaktionsende innerhalb von 16 h weitergerührt. Danach wird das Reaktionsgemisch auf 50° C abgekühlt, mit Wasser verdünnt und mit Natronlauge neutralisiert.

Anschließend wird das Gemisch mehrmals mit Toluol extrahiert. Die vereinigten organischen Phasen werden über Natriumsulfat getrocknet. Nach dem Entfernen des Lösungsmittels wird der Rückstand gaschromatografisch analysiert. Die Zusammensetzung der wäßrigen Phase wird durch Hochdruckflüssigkeitschromatographie bestimmt. In der nachstehenden Tabelle sind die auf diese Weise ermittelten Mengen an entstandenen Aminosulfonsäuren angegeben.

Beispiel 2 (Vergleichsbeispiel)

Es wurde wie in Beispiel 1 beschrieben gearbeitet, mit dem einzigen Unterschied, daß kein Ammoniumsulfat zugesetzt wurde.

Die auf diese Weise entstandenen Mengen an Aminosulfonsäuren sind ebenfalls in der nachstehenden Tabelle angegeben.

3

## Beispiel 3

In eine Mischung aus 561,5 g 96 %ige Schwefelsäure (5,5 Mol) und 132 g Ammoniumsulfat (1,0 Mol) werden unter Kühlung bei 25°C 143,0 g 1-Aminonaphthalin (1,0 Mol) in fester Form eingetragen. Anschließend wird das Gemisch innerhalb von 30 Minuten auf 110°C erwärmt und bei dieser Temperatur 16 Stunden weitergerührt. Danach wird auf Raumtemperature abgekühlt, das Reaktionsgemisch auf 700 g Eis ausgetragen und 6 h nachgerührt. Der Niederschlag wird abgesaugt, mit Wasser säurefrei gewaschen, gut abgepreßt und im Vakuum getrocknet.

Es werden 227.1 g einer 87.5 %igen p-Naphthionsäure erhalten: d.h. die Ausbeute beträgt 89,1 % der Theorie (bezogen auf eingesetztes 1-Aminonaphthalin).

Praktisch gleich Ausbeuten an p-Naphthionsäure wurden erhalten,wenn statt des Ammoniumsulfates die gleiche molare Menge an Natriumsulfat, Kaliumsulfat, Natriumbisulfat, Triethylamin oder Harnstoff einge-

## Tabelle

| Bsp. | $1\text{-}NH_2\text{-}6\text{-}SO_3H$ [Gew.-%] | $1\text{-}NH_2\text{-}5\text{-}SO_3H$ [Gew.-%] | $1\text{-}NH_2\text{-}4\text{-}SO_3H$ [Gew.-%] | $1\text{-}NH_2\text{-}7\text{-}SO_3H$ [Gew.-%] | $1\text{-}NH_2\text{-}3\text{-}SO_3H$ [Gew.-%] |
|------|------|------|------|------|------|
| 1) | 0.32 | 0.71 | 20.52 | 0.42 | - |
| 2) | 5.1 | 8.4 | 5.3 | 3.2 | 4.7 |

| Bsp. | $1\text{-}NH_2\text{-}4,7\text{-}SO_3H$ [Gew.-%] | $1\text{-}NH_2\text{-}2,4\text{-}SO_3H$ [Gew.-%] | $1\text{-}NH_2$ [Gew.-%] |
|------|------|------|------|
| 1) | 0.02 | 0.10 | 2.73 |
| 2) | 1.1 | 0.40 | 0.9 |

setzt wurden.

## Ansprüche

1. Verfahren zur Herstellung von 1-Aminonaphthalin-4-sulfonsäure durch Sulfonieren von 1-Aminonaphthalin, dadurch gekennzeichnet, daß man 1-Aminonaphthalin mit 3 bis 10 Mol 85-100 %iger Schwefelsäure je Mol 1-Aminonaphthalin bei Temperaturen von 40-160° C sulfoniert und die Sulfonierung in Gegenwart von Säureamiden oder von Alkali-, Erdalkali-oder Ammonium-sulfaten, -bisulfaten oder Alkali-, Erdalkali- oder Ammoniumsalzen von solchen Säuren vornimmt, die in Schwefelsäure aus ihren Salzen verdrängt werden, wobei man die Säureamide in einer Menge von 0,5-4 Mol je Mol 1-Aminonaphthalin und die Salze in einer Menge von 0,1-5 Äquivalenten je Mol 1-Aminonaphthalin anwendet.

2. Verfahren gemäß Anspruch 1, dadurch gekennzeichnet, daß man die Sulfonierung in Gegenwart von Alkali-, Erdalkali- oder Ammonium-sulfaten, -bisulfaten, -halogeniden, -phosphaten, -boraten, -carbonaten, -bicarbonaten, -sulfiten, -bisulfiten, -acetaten oder -naphthionaten vornimmt.

## Claims

1. Process for the preparation of 1-aminonaphthalene-4-sulphonic acid by sulphonation of 1-aminonaphthalene, characterised in that 1-aminonaphthalene is sulphonated at temperatures of 40-160° C using 3 to 10 mol of 85-100 % strength sulphuric acid per mol of 1-aminonaphthalene and the sulphonation is carried out in the presence of acid amides or of alkali metal, alkaline earth metal or ammonium sulphates or bisulphates, or alkali metal, alkaline earth metal or ammonium salts of those acids which are displaced from their salts in sulphuric acid, the acid amides being used in an amount of 0.5-4 mol per mol of 1-aminonaphthalene and the salts being used in an amount of 0.1-5 equivalents per mol of 1-aminonaphthalene.

2. Process according to Claim 1, characterised in that the sulphonation is carried out in the presence of alkali metal, alkaline earth metal or ammonium sulphates, bisulphates, halides, phosphates, borates, carbonates, bicarbonates, sulphites, bisulphites, acetates or naphthionates.

## Revendications

1. Procédé de préparation de l'acide 1-aminonaphtalène-4-sulfonique par sulfonation du 1-aminonaphtalène, caractérisé en ce que l'on sulfone le 1-aminonaphtalène par 3 à 10 mol d'acide sulfurique à 85-100 % par mole du 1-aminonaphtalène à des températures de 40 à 160° et on effectue la sulfonation en présence d'amides ou de sulfates ou bisulfates alcalins, alcalino-terreux ou d'ammonium ou de sels alcalins, alcalino-terreux ou d'ammonium d'acides qui sont déplacés de leurs sels par l'acide sulfurique, en utilisant les amides en quantités de 0,5 à 4 mol par mole du 1-aminonaphtalène et les sels en quantités de 0,1 à 5 équivalents par mole du 1-aminonaphtalène.

2. Procédé selon la revendication 1, caractérisé en ce que l'on effectue la sulfonation en présence de sulfates, bisulfates, halogénures, phosphates, borates, carbonates, bicarbonates, sulfites, bisulfites, acétates ou naphtionates alcalins, alcalinoterreux ou d'ammonium.